(19) Europäisches Patentamt
European Patent Office
Office européen des brevets

(11) EP 3 550 301 B1

(12) **EUROPEAN PATENT SPECIFICATION**

(45) Date of publication and mention of the grant of the patent:
**30.09.2020 Bulletin 2020/40**

(21) Application number: **18838024.0**

(22) Date of filing: **01.02.2018**

(51) Int Cl.:
***G01N 33/44*** *(2006.01)* ***C09J 133/08*** *(2006.01)*
***C09J 133/10*** *(2006.01)* ***C09J 133/12*** *(2006.01)*
***C09J 7/00*** *(2018.01)* ***G01N 19/10*** *(2006.01)*
***G01N 33/00*** *(2006.01)*

(86) International application number:
**PCT/KR2018/001373**

(87) International publication number:
**WO 2019/022330 (31.01.2019 Gazette 2019/05)**

(54) **METHOD FOR ANALYZING ACRYLIC ACID CONTENT IN ACRYLIC ADHESIVE RESIN COPOLYMER**

VERFAHREN ZUR ANALYSE DES ACRYLSÄUREGEHALTS IN ACRYLKLEBEHARZ-COPOLYMER

PROCÉDÉ D’ANALYSE DE LA TENEUR EN ACIDE ACRYLIQUE DANS UN COPOLYMÈRE DE RÉSINE ADHÉSIVE ACRYLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.07.2017 KR 20170096308**

(43) Date of publication of application:
**09.10.2019 Bulletin 2019/41**

(73) Proprietor: **LG Chem, Ltd.**
**Seoul 07336 (KR)**

(72) Inventors:
- **KIM, Dong Hyun**
  **Daejeon 34122 (KR)**
- **KIM, Byoung Hyoun**
  **Daejeon 34122 (KR)**
- **HAN, Su Youn**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
**JP-A- H0 933 508** **JP-A- 2003 270 225**
**JP-A- 2005 181 137** **JP-A- 2010 121 998**
**JP-A- 2016 210 929** **US-A1- 2016 009 960**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

TECHNICAL FIELD

This application claims the benefit of priority to Korean Patent Application No. 10-2017-0096308, filed on July 28,

The present invention relates to an analysis method of acrylic acid content in an acrylic copolymer adhesive resin.

BACKGROUND ART

It is known that the amount of acrylic acid in an acrylic copolymer adhesive resin is a main factor affecting the properties of adhesives (*see* Polymer Testing 27 (2008) 870: International Journal of Adhesion & Adhesives 34 (2012) 107). In order to analyze such an acrylic acid, the presence of acrylic acid have been confirmed by using FT-IR (Fourier transform infrared) or the content of acrylic acid have been analyzed by using GC (gas chromatography) (*see* Polymer Testing 27 (2008) 870). However, there were examples that the FT-IR exhibited the presence of acrylic acid even in the case that the acrylic acid does not contained. Also, there is a trial of using an EGA-GC/MS (Evolved Gas Analysis-Gas Chromatography/Mass Spectrometry) wherein an acrylic acid content is confirmed by measuring the amount of moisture generated from the acrylic polymer having carboxylic groups by a ring formation reaction under a high temperature environment. However, this is not proper in the analysis of acrylic acid content due to problems such as water obstruction and peak overlapping detected in the EGA-GC / MS itself.

Meanwhile, in the case of using an MASS (Moisture Analyzer for Solid Sample) for analyzing an acrylic acid content, it need to remove moisture from outside, and optimize a temperature condition that makes the completion of the ring formation reaction of carboxylic groups generating moisture and the amount of a sample that allows good heat transfer in the sample.

Document US2016/009960 calculates the water content of a pressure sensitive adhesive layer by the Karl Fisher method, using a Karl Fisher moisture meter at 230ºC.

The present inventors have endeavored to solve the above problems and found that the amount of moisture generated from the acrylic polymer having carboxylic groups by a ring formation reaction under a high temperature environment is measured by using a moisture analyzer for solid sample (MASS), and then the acrylic acid content can be analyzed based on the measured amount of moisture and the used amount of the sample.

DETAILED DESCRIPTION OF THE INVENTION

Technical Problem

It is an aspect of the present invention to provide an analysis method of an acrylic acid content, which uses to generate the ring formation reaction of an acrylic polymer having carboxylic groups under a high temperature environment.

Technical Solution

An acrylic polymer having carboxylic groups generates moisture during the ring formation reaction of the carboxylic groups under a high temperature environment as follows:

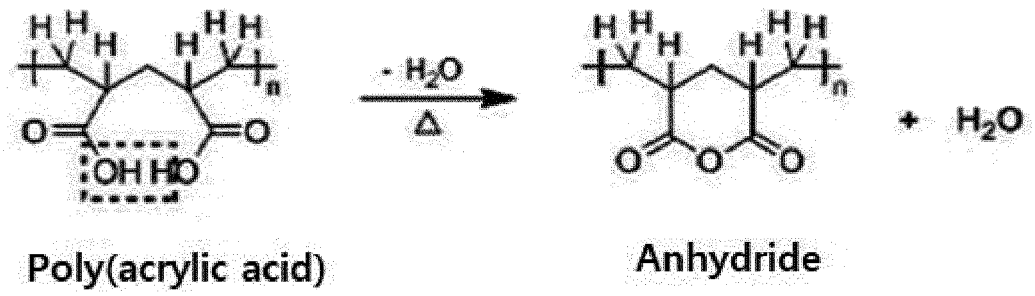

Poly(acrylic acid)
Anhydride

The present invention measures the amount of moisture generated during the ring formation reaction of the carboxylic groups using an MASS which is self-manufactured and calculates an acrylic acid content from the measurement, and the calculated acrylic acid content is confirmed to correspond with the results of $^1$H HR MAS NMR experiments.

In order to accomplish the technical solution, the present invention provides analysis method of an acrylic acid content by measuring the amount of moisture generated from an acrylic copolymer adhesive resin using an MASS and

calculates an acrylic acid content based on the measured amount of moisture and the used amount of the sample.

The present invention provides an analysis method of an acrylic acid content in an acrylic copolymer adhesive resin, comprising measuring the amount of moisture generated from the acrylic copolymer adhesive resin obtained by polymerization of two or more acrylic acid monomers using a moisture analyzer for solid sample (MASS), and putting the measurement into the following Equation 1 to calculate an acrylic acid content:

[Equation 1]

$$\text{AA Content (\%)} = \frac{W_{H2O} \times 2 \times MW_{AA}}{1000 \times MW_{H2O} \times W_S} \times 100$$

wherein,

AA represents an acrylic acid,
$W_{H2O}$ is the measured amount (μg) of moisture,
the value of "2" in the numerator represents that 1 mole of $H_2O$ is generated per 2 moles of acrylic acids,
$MW_{AA}$ is the molecular weight (72.06 g/mol) of acrylic acid monomer,
the value of "1000" in the denominator is used for the calibration of a weight unit,
$MW_{H2O}$ is the molecular weight (18.02 g/mol) of $H_2O$, and
$W_S$ is the amount (mg) of a sample.
In the present invention, the amount of moisture is measured by using the MASS at a final temperature ranging from 355 to 365°C. If the final temperature of the MASS measurement is 350°C, the ring formation reaction is insufficiently carried out and the amount of moisture is measured to be low. If the final temperature is 370°C, moisture is further generated by a ring cleavage reaction after ring formation and the moisture value is evaluated more than the actual amount.

In one embodiment, the two or more acrylic acid monomers comprise two or more acrylic acids selected from the group consisting of acrylic acid (AA), ethyl hexyl acrylate (EHA), butyl acrylate (BA), methyl acrylate (MA), ethyl acrylate (EA), ethyl hexyl methacrylate (EHMA), butyl methacrylate (BMA), methyl methacrylate (MMA) and ethyl methacrylate (EMA).

In one embodiment, the two or more acrylic acid monomers comprise two or more acrylic acids selected from the group consisting of acrylic acid (AA), ethyl hexyl acrylate (EHA) and butyl acrylate (BA).

In one embodiment, the acrylic copolymer adhesive resin is made in the form of a film by polymerization of the monomers, a solvent and a thermal radical initiator (TRI).

In one embodiment, the solvent comprises ethyl acetate (EtOAc) or solvents having a boiling point of 60 to 78°C.

In one embodiment, the TRI comprises azobis(isobutyronitrile) (AIBN) or diazo compounds.

In one embodiment, the polymerization is carried out at a temperature of 60 to 78°C.

## Advantageous Effects

The quantitative analysis method of an acrylic acid content according to the present invention can provide a reproducible analysis value of acrylic acid content corresponding with the results of NMR experiments, while avoiding demerits of the conventional methods.

## BEST MODE

Hereinafter, the present invention will be described in detail.

It should be understood that the terms used in the specification and the appended claims should not be construed as limited to general and dictionary meanings, but interpreted based on the meanings and concepts corresponding to technical aspects of the present invention on the basis of the principle that the inventor is allowed to define terms appropriately for the best explanation.

The analysis method of an acrylic acid content according to the present invention comprises measuring the amount of moisture generated from the acrylic copolymer adhesive resin obtained by polymerization of two or more acrylic acid monomers by using a moisture analyzer for solid sample (MASS), and putting the measurement into the following Equation 1 to calculate an acrylic acid content:

[Equation 1]

$$\text{AA Content (\%)} = \frac{W_{H2O} \times 2 \times MW_{AA}}{1000 \times MW_{H2O} \times W_S} \times 100$$

wherein,

AA represents an acrylic acid,
$W_{H2O}$ is the measured amount ($\mu$g) of moisture,
the value of "2" in the numerator represents that 1 mole of $H_2O$ is generated per 2 moles of acrylic acids,
$MW_{AA}$ is the molecular weight (72.06 g/mol) of acrylic acid monomer,
the value of "1000" in the denominator is used for the calibration of a weight unit,
$MW_{H2O}$ is the molecular weight (18.02 g/mol) of $H_2O$, and
$W_S$ is the amount (mg) of a sample.

The amount of moisture is measured by using the MASS at a final temperature ranging from 355 to 365°C. If the final temperature of the MASS measurement is 350°C, the ring formation reaction is insufficiently carried out and the amount of moisture is measured to be low. If the final temperature is 370°C, moisture is further generated by a ring cleavage reaction after ring formation and the moisture value is evaluated more than the actual amount.

In one embodiment, the two or more acrylic acid monomers comprise two or more acrylic acids selected from the group consisting of acrylic acid (AA), ethyl hexyl acrylate (EHA), butyl acrylate (BA), methyl acrylate (MA), ethyl acrylate (EA), ethyl hexyl methacrylate (EHMA), butyl methacrylate (BMA), methyl methacrylate (MMA) and ethyl methacrylate (EMA), for example two or more acrylic acids selected from the group consisting of acrylic acid (AA), ethyl hexyl acrylate (EHA) and butyl acrylate (BA).

In one embodiment, the acrylic copolymer adhesive resin is made in the form of a film by polymerization of the monomers, a solvent and a thermal radical initiator (TRI).

In one embodiment, the solvent comprises ethyl acetate (EtOAc) or solvents having a boiling point of 60 to 78°C.

In one embodiment, the TRI comprises azobis(isobutyronitrile) (AIBN) or diazo compounds.

In one embodiment, the polymerization is carried out at a temperature of 60 to 78°C.

The acrylic acid content analyzed by the quantitative analysis method according to the present invention is well matched with the used amount of each monomer and is substantially corresponded with the results of $^1$H HR MAS NMR experiments.

Hereinafter, the present invention will be described in more detail with reference to Examples. It will be apparent to those skilled in the art that the following examples are intended to be illustrative of the present invention and not to be construed as limiting the scope of the invention.

## Example

### Preparation of acrylic copolymer adhesive resin sample

As listed in Table 1, 6 acrylic adhesive resin samples in which the type and content ratio of monomers (wt/wt) were each different, i.e., B99A1 (BA 99%/AA 1%), B95A5 (BA 95%/AA 5%), B90A10 (BA 90%/AA 10%), E99A1 (EHA 99%/AA 1%), E95A5 (EHA 95%/AA 5%) and E90A10 (EHA 90%/AA 10%) were prepared by adding the corresponding monomers in EtOAc as s solvent for 1 hour, to which AIBN is added as a TRI, followed by polymerization. The polymerization was carried out 78°C, the boiling point of EtOAc for 6 hours to give a copolymer conversion of 99.7%. After polymerization, the resulting copolymer was added with a solvent of EtOAc to control its viscosity (1000 to 2000 cP) which is suitable for coating, thereby obtaining a coating solution for forming a film. After coating, drying was carried to remove the solvent. The coating solution generally had a total solid content (TSC) of 10% to 20%.

In the coating procedure, the copolymer solution was coated on the surface of a release film in a thickness of 25 $\mu$m by using a bar coater and dried in an oven (convection oven) set to 120°C for 3 minutes to remove the solvent EtOAc and the unreacted monomer. After drying, another release film having different peel strength was laminated on the coated surface to form a film having the release films on both sides.

[Table 1] Monomer Composition of 6 acrylic adhesive resin samples

| Acrylic Adhesive | wt% | | |
|---|---|---|---|
| | 1)BA | 2)EHA | 3)AA |
| B99A1 | 99 | 0 | 1 |
| B95A5 | 95 | 0 | 5 |
| B90A10 | 90 | 0 | 10 |
| E99A1 | 0 | 99 | 1 |
| E95A5 | 0 | 95 | 5 |
| E90A10 | 0 | 90 | 10 |

1)BA: Butyl Acrylate
2)EHA: 2-Ethyl Hexyl Acrylate
3)AA: Acrylic Acid

## Analysis Method and Conditions

(1) EGA-MS(Evolved Gas Analysis-Mass Spectrometry) Analysis

EGA-MS analysis was carried out to confirm the temperature range in which moisture was generated from the acrylic adhesive. The apparatus used for the EGA-MS analysis was a double-shot pyrolyzer (PY-2020id) manufactured by Frontier Laboratories and GC/MSD (Agilent 7890A GC system/5975C inert XL mass selective detector) equipped with ALLOY-DTM (deactivated metal column) (0.15 mm (I.D.) x 2.5 m (L), coated film thickness < 0.01 μm). The pyrolyzer was maintained at 50°C for 5 minutes and its temperature was raised to 600°C by the rise rate of 10°C per minute to give a pyrogram. The flow rate of a carrier gas (He) was 1.0 mL/min and the split ratio was 20:1. The temperature of an injector, an oven and an interface in the GC/MSD was 300°C, the temperature of the pyrolyzer interface was 320°C, and the scan range was from 15 to 700 amu.

(2) $^1$H HR MAS NMR (High Resolution Magic Angle Spinning Nuclear Magnetic Resonance) Analysis

This analysis was performed using the apparatus of Agilent 600MHz SSNMR (Solid State Nuclear Magnetic Resonance) equipped with a NANO probe at room temperature (25°C). $^1$H HR MAS experiments were carried out by using an s2pul pulse sequence under the conditions of scan number = 16, relaxation delay = 5 seconds, pulse width = 9.5 μsec, acquisition time = 1.7 seconds, and spinning rate = 2.3 kHz. The samples were loaded on $CDCl_3$ in a $^1$H HR MAS rotor and swelled for 3 hrs or more before measurement.

(3) Karl Fischer (K/F) Titrator

The K/F titrator was used with HYDRANAL-Coulomat AG-Oven (Sigma-Aldrich, Cat. No. 34739-500ML-R) as a reagent. The extraction time of the K/F titrator was set to 300 seconds, the start drift value was set to 20 μg/min, the stop time was set to OFF, and the initial drift value was 10 μg/min or less.

(4) Measurement of Moisture Amount in Standard Material Using MASS (Moisture Analyzer for Solid Sample)

1% K/F oven standard ($Na_2WO_4 \cdot 2H_2O$) was used as a standard material and the flow rate of helium was set to 100 mL/min. The temperature condition had two intervals. The first temperature interval was maintained at 50°C for 5 minutes, and the valve was set to the OFF state to purge the vaporized substances. The second temperature interval was set to raise from 50°C to 450°C for 35 minutes, and the valve was set to the ON state so that the vaporized substances were introduced into the K/F titrator.

(5) Measurement of Moisture Amount in Sample Using MASS (Moisture Analyzer for Solid Sample)

Similar to the measurement of moisture amount in standard material, the flow rate of helium was set to 100 mL/min. The temperature condition had four intervals. The first and second temperature intervals were set to raise from

room temperature to 130°C for 5 minutes and maintained at 130°C for 5 minutes, and the valve was set to the OFF state in the two intervals to purge the vaporized substances. The third and fourth temperature intervals were set to raise from 130°C to 360°C for 5 minutes and maintained at 360°C for 10 minutes, and the valve was set to the ON state in the two intervals so that the vaporized substances were introduced into the K/F titrator.

The aliquots of the samples in the amount of 15 to 25 mg were exactly weighed in the unit of 0.1 mg and applied to the experiments. The content of an acrylic acid in the samples was calculated by using the content ($\mu$g) of moisture measured via the K/F titrator.

**Calculation of Acrylic Acid Content**

The acrylic acid content in the acrylic adhesive resin samples was calculated by measuring the amounts of the acrylic adhesive resin samples and the amount of moisture generated therefrom by using the MASS, and putting the measurements into the following Equation 1:

[Equation 1]

$$\text{AA Content (\%)} = \frac{W_{H2O} \times 2 \times MW_{AA}}{1000 \times MW_{H2O} \times W_S} \times 100$$

wherein,

AA represents an acrylic acid,
$W_{H2O}$ is the measured amount ($\mu$g) of moisture,
the value of "2" in the numerator represents that 1 mole of $H_2O$ is generated per 2 moles of acrylic acids,
$MW_{AA}$ is the molecular weight (72.06 g/mol) of acrylic acid monomer,
the value of "1000" in the denominator is used for the calibration of a weight unit,
$MW_{H2O}$ is the molecular weight (18.02 g/mol) of $H_2O$, and
$W_S$ is the amount (mg) of a sample.

The amount of moisture was measured by using the MASS at a final temperature ranging from 355 to 365°C.

Analysis Results

(1) MASS Analysis

The results of acrylic acid content in the acrylic adhesive resin samples measured by using the MASS are shown in Table 2 below.

From the RSD values of Table 2 below, it was confirmed that all of 6 acrylic adhesive resin samples exhibited reproducible results. In the acrylic acid content ("Average"), BA-based samples (B99A1, B95A5 and B90A10) were well matched with the feed of the monomers used for their preparation, while EHA-based samples (E99A1, E95A5 and E90A10) exhibited a difference of 0.3% to 0.5% as compared with the feed of the monomers.

[Table 2] Analysis Results of Acrylic Acid Content by Mass

| Sample | Feed (AA, wt%) | Results (AA, wt%) | Average (n=3) | RSD (%) |
|---|---|---|---|---|
| B99A1 | 1.0 | 0.9 | 1.0 | 15.9 |
| | | 1.0 | | |
| | | 1.2 | | |
| B95A5 | 5.0 | 5.0 | 5.0 | 1.2 |
| | | 4.9 | | |
| | | 5.0 | | |

(continued)

| Sample | Feed (AA, wt%) | Results (AA, wt%) | Average (n=3) | RSD (%) |
|---|---|---|---|---|
| B90A10 | 10.0 | 10.3 | 10.0 | 2.8 |
| | | 10.1 | | |
| | | 9.7 | | |
| E99A1 | 1.0 | 1.3 | 1.3 | 0.0 |
| | | 1.3 | | |
| | | 1.3 | | |
| E95A5 | 5.0 | 4.7 | 4.5 | 4.0 |
| | | 4.3 | | |
| | | 4.6 | | |
| E90A10 | 10.0 | 10.3 | 10.4 | 0.5 |
| | | 10.4 | | |
| | | 10.4 | | |

(2) $^1$H HR MAS NMR Analysis

$^1$H HR MAS NMR analysis was performed in order to confirm the accuracy of the acrylic acid content values in the acrylic adhesive resin samples as shown in Table 2 above, which were obtained from the MASS experiment. The results thereof are shown in Table 3 below.

Comparing the MASS results shown in Table 2 above with the $^1$H HR MAS NMR results shown in Table 3 below regarding the acrylic acid content, the 5 samples excluding E95A5 (EHA 95%/AA 5%) exhibited a difference within 0.5%, i.e., 0.1%, 0.2% or 0.4%, while E95A5 (EHA 95%/AA 5%) exhibited a difference of 1.0%, specifically 4.5 wt% (MASS) and 5.5 wt% (NMR). It is understood that there was an error due to moisture peaks detected during the $^1$H HR MAS NMR experiment.

[Table 3] Analysis Results of Acrylic Acid Content by $^1$H HR MAS NMR

| Sample | Feed (AA, wt%) | Content ratio (wt/wt) by $^1$H HR MAS NMR | | |
|---|---|---|---|---|
| | | AA | BA | EHA |
| B99A1 | 1.0 | 1.1 | 98.9 | - |
| B95A5 | 5.0 | 5.2 | 94.8 | - |
| B90A10 | 10.0 | 10.4 | 89.6 | - |
| E99A1 | 1.0 | 1.2 | - | 98.8 |
| E95A5 | 5.0 | 5.5 | - | 94.5 |
| E90A10 | 10.0 | 10.9 | - | 89.1 |

From the MASS results shown in Table 2 above with the $^1$H HR MAS NMR results shown in Table 3 above regarding the acrylic acid content, it was confirmed that the present invention can provide reproducible analysis values of acrylic acid content by measuring the amount of moisture generated from the acrylic copolymer adhesive resin by using the MASS, followed by calculation putting the measured amount of moisture and the used amount of the acrylic copolymer adhesive resin into Equation 1, and the obtained content was corresponded with the results of NMR experiments.

## Claims

**1.** An analysis method of an acrylic acid content in an acrylic adhesive resin copolymer, comprising measuring the

amount of moisture generated from the acrylic adhesive resin copolymer obtained by polymerization of two or more acrylic acid-based monomers by using a moisture analyzer for solid sample and calculate the content of the acrylic acid by inserting the measurement into the following Equation 1:

[Equation 1]

$$\text{AA Content (\%)} = \frac{W_{H2O} \times 2 \times MW_{AA}}{1000 \times MW_{H2O} \times W_S} \times 100$$

wherein,

AA represents the acrylic acid,
$W_{H2O}$ is a measured amount in μg of moisture,
"2" in a numerator representing that 1 mole of $H_2O$ is generated per 2 moles of acrylic acids,
$MW_{AA}$ is the molecular weight of acrylic acid being 72.06 g/mol, "1000" in a denominator is for calibration of a weight unit,
$MW_{H2O}$ is the molecular weight of $H_2O$ being 18.02 g/mol, and
$W_S$ is an amount in mg of a sample of the acrylic copolymer adhesive resin,
wherein a final temperature at which the amount of moisture is measured by using the moisture analyser for solid sample is 355 to 365°C.

2. The analysis method of claim 1, wherein the two or more acrylic acid-based monomers comprise acrylic acid (AA), ethyl hexyl acrylate (EHA), butyl acrylate (BA), methyl acrylate (MA), ethyl acrylate (EA), ethyl hexyl methacrylate (EHMA), butyl methacrylate (BMA), methyl methacrylate (MMA) or ethyl methacrylate (EMA).

3. The analysis method of claim 2, wherein the two or more acrylic acid-based monomers comprise acrylic acid (AA), ethyl hexyl acrylate (EHA) or butyl acrylate (BA).

4. The analysis method of claim 1, wherein the acrylic adhesive resin copolymer is made in the form of a film by polymerization of the monomers, a solvent and a thermal radical initiator.

5. The analysis method of claim 4, wherein the solvent comprises ethyl acetate (EtOAc) or solvents having a boiling point of 60 to 78°C.

6. The analysis method of claim 4, wherein the thermal radical initiator comprises azobis(isobutyronitrile) (AIBN) or diazo compounds.

7. The analysis method of claim 4, wherein the polymerization is carried out at the boiling point of the solvent.

8. The analysis method of claim 7, wherein the polymerization is carried out at a temperature of 60 to 78°C.

## Patentansprüche

1. Analyseverfahren eines Acrylsäuregehalts in einem Acrylklebstoffharz-Copolymer, bei dem die Menge an Feuchtigkeit, die aus dem Acrylklebstoffharz-Copolymer erzeugt wird, das durch Polymerisation von zwei oder mehr Monomeren auf Acrylsäurebasis erhalten wird, unter Verwendung eines Feuchtigkeitsanalysators für feste Proben gemessen wird, und der Gehalt der Acrylsäure durch Einsetzen der Messung in die folgende Gleichung 1 berechnet wird:

[Gleichung 1]

$$\textit{Acrylsäure Gehalt}\ (\%) = \frac{W_{H2O} \times 2 \times MW_{AA}}{1000 \times MW_{H2O} \times W_S} \times 100,$$

wobei

AA für die Acrylsäure steht,
$W_{H2O}$ eine gemessene Menge Feuchtigkeit in µg ist,
"2" im Zähler darstellt, dass 1 Mol $H_2O$ pro 2 Mol Acrylsäuren erzeugt wird, $MW_{AA}$ das Molekulargewicht von Acrylsäure ist, das 72,06 g/mol beträgt,
"1000" im Nenner für die Kalibrierung einer Gewichtseinheit dient,
$MW_{H2O}$ das Molekulargewicht von $H_2O$ ist, das 18,02 g/mol beträgt, und
Ws eine Menge einer Probe des Acryl-Copolymer-Klebstoffharzes in mg ist,
wobei eine Endtemperatur, bei der die Feuchtigkeitsmenge unter Verwendung des Feuchtigkeitsanalysators für feste Proben gemessen wird, 355 bis 365°C beträgt.

**2.** Analyseverfahren nach Anspruch 1, bei dem die zwei oder mehr Monomere auf Acrylsäurebasis Acrylsäure (AA), Ethylhexylacrylat (EHA), Butylacrylat (BA), Methylacrylat (MA), Ethylacrylat (EA), Ethylhexylmethacrylat (EHMA), Butylmethacrylat (BMA), Methylmethacrylat (MMA) oder Ethylmethacrylat (EMA) umfassen.

**3.** Analyseverfahren nach Anspruch 2, bei dem die zwei oder mehr Monomere auf Acrylsäurebasis Acrylsäure (AA), Ethylhexylacrylat (EHA) oder Butylacrylat (BA) umfassen.

**4.** Analyseverfahren nach Anspruch 1, bei dem das Acrylklebstoffharz-Copolymer in Form eines Films durch Polymerisation der Monomere, eines Lösungsmittels und eines thermischen Radikalinitiators hergestellt wird.

**5.** Analyseverfahren nach Anspruch 4, bei dem das Lösungsmittel Ethylacetat (EtOAc) oder Lösungsmittel mit einem Siedepunkt von 60 bis 78°C umfasst.

**6.** Analyseverfahren nach Anspruch 4, bei dem der thermische Radikalinitiator Azobis(isobutyronitril) (AIBN) oder Diazoverbindungen umfasst.

**7.** Analyseverfahren nach Anspruch 4, bei dem die Polymerisation am Siedepunkt des Lösungsmittels durchgeführt wird.

**8.** Analyseverfahren nach Anspruch 7, bei dem die Polymerisation bei einer Temperatur von 60 bis 78°C durchgeführt wird.

## Revendications

**1.** Procédé d'analyse de la teneur en acide acrylique d'un copolymère de résine adhésive acrylique comprenant la mesure de la quantité d'humidité générée par le copolymère de résine adhésive acrylique obtenu par polymérisation de deux ou de plusieurs monomères à base d'acide acrylique en utilisant un analyseur d'humidité pour échantillon solide et par calcul de la teneur en acide acrylique en insérant la mesure dans l'équation suivante 1 :

[Équation 1]

$$\text{AA Content (\%)} = \frac{W_{H2O} \times 2 \times MW_{AA}}{1000 \times MW_{H2O} \times W_S} \times 100$$

AA Content (%) : Teneur en AA (%)
où,

AA représente l'acide acrylique,
$W_{H20}$ représente une quantité d'humidité mesurée en µg,
"2" désigne un numérateur indiquant qu'une mole de $H_2O$ est générée par 2 moles d'acides acryliques,
$MW_{AA}$ désigne le poids moléculaire de l'acide acrylique qui est de 72,06 g/mol,
"1000" dans un dénominateur sert à l'étalonnage d'une unité de poids,

$MW_{H20}$ désigne le poids moléculaire de $H_2O$ qui est de 18,02 g/mole, et
$W_S$ représente une quantité en mg d'un échantillon de la résine adhésive du copolymère acrylique,
dans laquelle la température finale à laquelle la quantité d'humidité mesurée en utilisant l'analyseur d'humidité pour échantillon solide est de 355 à 365 °C.

**2.** Procédé d'analyse selon la revendication 1, dans lequel les deux ou plusieurs monomères à base d'acide acrylique comprennent l'acide acrylique (AA), l'acrylate d'éthylhexyle (EHA), l'acrylate de butyle (BA), l'acrylate de méthyle (MA), l'acrylate d'éthyle (EA), le méthacrylate d'éthylhexyle (EHMA), le méthacrylate de butyle (BMA), le méthacrylate de méthyle (MMA) ou le méthacrylate d'éthyle (EMA).

**3.** Procédé d'analyse selon la revendication 2, dans lequel les deux ou plusieurs monomères à base d'acide acrylique comprennent l'acide acrylique (AA), l'acrylate d'éthylhexyle (EHA) ou l'acrylate de butyle (BA).

**4.** Procédé d'analyse selon la revendication 1, dans lequel le copolymère de résine adhésive d'acrylate est fabriqué sous la forme d'un film par polymérisation des monomères, d'un solvant et d'un initiateur de radicaux thermiques.

**5.** Procédé d'analyse selon la revendication 4, dans lequel le solvant comprend de l'acétate d'éthyle (EtOAc) ou des solvants ayant un point d'ébullition de 60 à 78 °C.

**6.** Procédé d'analyse selon la revendication 4, dans lequel l'initiateur de radicaux thermiques comprend des composés azobis(isobutyronitrile) (AIBN) ou diazoïques.

**7.** Procédé d'analyse selon la revendication 4, dans lequel la polymérisation est effectuée au point d'ébullition du solvant.

**8.** Procédé d'analyse selon la revendication 7, dans lequel la polymérisation est conduite à une température de 60 à 78 °C.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020170096308
- US 2016009960 A


### Non-patent literature cited in the description


- *Polymer Testing,* 2008, vol. 27, 870
- *International Journal of Adhesion & Adhesives,* 2012, vol. 34, 107